# EUROPEAN PATENT SPECIFICATION

(11) **EP 1 061 947 B1**
(45) Date of publication and mention of the grant of the patent: **16.06.2004**
(21) Application number: 99906095.7
(22) Date of filing: 08.03.1999
(51) Int. Cl.: A61K 38/26, A61P 3/10, A61P 3/04

(54) **STABILIZED AQUEOUS GLUCAGON SOLUTIONS COMPRISING DETERGENTS**
STABILISIERTE WÄSSERIGE GLUKAGONLÖSUNGEN ENTHALTEND DETERGENZIEN
SOLUTIONS AQUEUSES STABILISEES DE GLUCAGON COMPRENANT DES DETERGENTS

(30) Priority: 13.03.1998 EP 98610006
(43) Date of publication of application: 27.12.2000
(73) Proprietor: NOVO NORDISK A/S, 2880 Bagsvaerd (DK)
(72) Inventor: KAARSHOLM, Niels, C., DK-2720 Vanlose (DK)
(74) Representative: Plougmann & Vingtoft A/S
(86) International application number: PCT/DK1999/000115
(87) International publication number: WO 1999/047160

(56) References cited:
- WO-A-95/32730
- GB-A- 1 202 607
- R.M. EPAND ET AL.: "MOLECULAR INTERACTIONS IN THE MODEL LIPOPROTEIN COMPLEX FORMED BETWEEN GLUCAGON AND DIMYRISTOYLGLYCEROPHOSPHOCHOLINE." BIOCHEMISTRY., vol. 16, no. 20, 1997, pages 4360-4368, XP002074758 EASTON, PA US
- C. BÖSCH ET AL.: "PHYSICOCHEMICAL CHARACTERIZATION OF GLUCAGON-CONTAINING LIPID MICELLES." BIOCHIMICA ET BIOPHYSICA ACTA, vol. 603, no. 2, 12 December 1980, pages 298-312, XP002074759 AMSTERDAM, NL cited in the application

## Description

### FIELD OF THE INVENTION

The present invention relates to stable, injectable aqueous solutions of peptides such as glucagon, glucagon-like peptide-1 (GLP-1, e.g. GLP-1 (7-37) and GLP-1 (7-36) amide) and analogues and derivatives thereof.

### BACKGROUND OF THE INVENTION

Glucagon and glucagon-like peptide 1 (GLP-1) are polypeptide hormones both derived from proglucagon. Upon tissue-specific processing, glucagon is produced in the pancreas, while GLP-1 is predominantly secreted in the intestine. Physiologically, both peptides play major roles in the regulation of blood glucose. Glucagon is directly involved via glycogenolytic as well as gluconeogenetic effects. GLP-1 is indirectly involved via stimulation of insulin and inhibition of glucagon secretion. Thus, the two peptides affect the blood glucose in opposite directions.

Due to its glycogenolytic effect on the liver, glucagon is used for the treatment of acute hypoglycemia (e.g. "insulin shock"). In addition, glucagon excerts a spasmolytic effect on smooth muscles, and this effect is used clinically in connection with several imaging procedures, especially radiology.

GLP-1 fragments such as GLP-1(7-37), GLP-1 (7-36) amide and functional analogues thereof (hereinafter designated GLP-1 compounds) stimulate pancreatic insulin release in the presence of glucose, and this finding suggests that GLP-1 compounds may be useful in the treatment of type II diabetes.

Glucagon (29 amino acids; see e.g. *The Merck Index*, 10^{th} Edition for the amino acid sequence) and GLP-1 compounds (typically about 29-31 amino acids) are single chain highly homologous peptides. While little information is available on the solution behaviour of GLP-1 compounds, the physico-chemical properties of glucagon are well described in the literature (J.Biol.Chem. 244, 6675-6679 (1969); Nature 257, 751-757 (1975); FEBS Letts. 119, 265-270 (1980)). The peptide exhibits very low solubility in water at a pH in the range of about 4-9. Outside this region, aqueous solutions are generally metastable and prone to gel formation or flocculation within hours of their preparation.

Accordingly, glucagon compositions for injection are currently marketed in the form of a lyophilized powder which is to be reconstituted using a suitable diluent shortly before use. The resulting acidic solution may then be injected. In contrast with the single-dose compositions of glucagon used for acute hypoglycemia and the motility indication in radiology, the utility of GLP-1 compounds for the treatment of type II diabetes generally involves multiple-dose soluble compositions. Given the instability of glucagon, the formulation of such compositions is inherently difficult.

Thus, there is a need for stable aqueous formulations of peptides such as glucagon and GLP-1 compounds. In the case of glucagon, the availability of a ready-to-use composition is desirable for emergency treatment of acute hypoglycemia, enabling the patient or another person present to treat the hypoglycemic incidence immediately. Also for the mobility indication for glucagon used in radiology, and for the type II diabetes indication for GLP-1 compounds, the availability of a ready-to-use aqueous composition provides obvious advantages in terms of convenience and safety.

Glucagon compositions solubilized by the addition of detergent have received relatively little attention in the literature. British patent No. 1 202 607 describes the use of either cationic (quarternary ammonium bases) or anionic (alkyl-aryl-sulphonates). monovalent detergents. These compounds provide enhanced solubilization of 1 mg/ml glucagon over a limited pH range using a 6-fold molar excess of detergent.

A series of other studies on glucagon·detergent interactions employ much lower glucagon concentrations and do not address the effect of detergent on peptide solubility. Thus, in studies using 0-200 µM of the amphoteric (twitterionic) detergent hen egg lysolecithin, binding of detergent was shown to induce partial helical structure in solutions of glucagon containing about 0.02 mg/ml peptide (J.Biol.Chem. 247. 4986-4991; 4992-4995; (1972)). Similarly, in studies employing dilute (0.005-0.05 mg/ml) solutions of glucagon, lysolecithin was found more efficient than the anionic detergents dipalmitoyl phosphatidic acid and sodium dodecyl sulfate (SDS) in inducing partial helical structure in glucagon (Biopolymers 21, 1217-1228 (1982); Biopolymers 22, 1003-1021 (1983)).

At the other extreme, a few examples are available characterizing the interaction between glucagon and concentrated detergent solutions. These include the binding of dilute glucagon to micelles of the anionic detergent SDS at 25 mM, pH 2 (Biochemistry 19, 2117-2122, (1980)), and the solubilization of glucagon (up to 20 mg/ml) via binding to micelles of the amphoteric dodecylphosphocholine present in a 40-fold molar excess (e.g. 3.5 mg/ml glucagon is solubilized using 40 mM detergent at neutral pH; Biochim.Biophys.Acta 603, 298-312, (1980))

With the exception of a single example describing the solubilization of 20 mg/ml GLP-1 (7-36) amide bound to micelles of dodecylphosphocholine at 280 mM (cf. the glucagon case above, Biochemistry 33, 3532-3539, 1994), no reports are available on the binding of detergents by GLP-1 compounds and the resulting effect on peptide solubility and stability. In contrast, several ways of preparing prolonged delivery of GLP-1 compounds have been described (e.g. European patent application No. 0619322 and column 6 of US patent No. 5, 120, 712). The possibilities include use of polymers, incorporation into particles of polymer material or into an oil suspension, addition of zinc ion, protamine sulphate and/or phenolic compounds, and preparation of amorphous/crystalline formulations.

### BRIEF DESCRIPTION OF THE INVENTION

It has now surprisingly been found that soluble and stable formulations of glucagon and GLP-1 compounds may be prepared by means of addition of detergents according to claim 1 carrying multiple charges (i.e. two or more negative, two or more positive, or both positive and negative charges). When added in an amount corresponding to e.g. about 0.5-20 moles of detergent per mole of peptide, this class of detergents is capable of solubilizing pharmaceutically relevant concentrations of glucagon and GLP-1 compounds in the entire pH range from 4 to 9. The expanded pH range allows optimal selection of conditions to suppress chemical degradation of the solubilized peptide. Furthermore, preferred detergents consist of naturally occurring building blocks which are more biologically acceptable than those previously described.

The invention relates to an aqueous composition in the form of a solution comprising at least one peptide selected from glucagon, GLP-1, and analogues and derivatives thereof together with a stabilizing and solubilizing amount of at least one detergent as defined in the claims, said detergent having at least 2 positive charges, at least 2 negative charges, or a combination of at least one positive charge and at least one negative charge, the peptide or analogue or derivative thereof being present in a concentration of at least about 0.1 mg/ml, with the proviso that the detergent is not dodecyl phosphocholine.

Another aspect of the invention relates to a method for stabilizing an aqueous composition in the form of a solution comprising at least one peptide selected from glucagon, GLP-1 and analogues and derivatives thereof by adding to the composition a stabilizing and solubilizing amount of at least one such detergent as defined in the claims having multiple charges.

In a further embodiment, the invention relates to the provision of a medicament for treating insulin dependent or non-insulin dependent diabetes mellitus or obesity in a patient in need of such treatment, comprising administering to the patient a therapeutically effective amount of an appropriate stabilized aqueous composition as defined in the claims.

### DETAILED DESCRIPTION OF THE INVENTION

Glucagon and GLP-1 compounds belong to a class of polypeptides which have the potential of forming amphiphatic helices, i.e., one face of the helical structure displays mainly hydrophobic amino acid side chains, while the other face exposes hydrophilic and:or charged side chains. In polypeptides of this type, the formation of the amphiphatic helical structure is often linked to inter-peptide helix self-association along the interfaces defined by the hydrophobic patches.

A simple system is used to describe fragments and analogues of GLP-1. Thus, for example, Gly⁸-GLP-1 (7-37) designates a fragment of GLP-1 formally derived from GLP-1 by deleting the amino acid residues Nos. 1 to 6 and substituting the naturally occurring amino acid residue in position 8 (Ala) by Gly. Similarly, Lys³⁴(N^{ε}-tetradecanoyl)-GLP-1(7-37) designates GLP-1(7-37) wherein the ε-amino group of the Lys residue in position 34 has been tetradecanoylated.

The amino acid sequence of GLP-1 (7-36)amide and GLP-1 (7-37) is as given in the general formula (I) below: 
7 8 9 10 11 12 13 14 15 16 17 18 19 20 21 2[?] 23
His-Ala-Glu-Gly-Thr-Phe-Thr-Ser-Asp-Val-Ser-Ser-Tyr-Leu-Glu-Gly-Gln-

24 25 26 27 28 29 30 31 32 33 34 35 36 (I)
Ala-Ala-Lys-Glu-Phe-Ile-Ala-Trp-Leu-Val-Lys-Gly-Arg-X
 which shows GLP-1(7-36)amide when X is NH₂ and GLP-1 (7-37) when X is Gly-OH.

In the present text, the designation "analogue" is used to designate a peptide wherein one or more amino acid residues of the basic GLP-1 peptide have been substituted by another amino acid residue and/or wherein one or more amino acid residues of the peptide have been deleted and/or wherein one or more amino acid residues have been added to the peptide. Such addition can take place either at the N-terminal end or at the C-terminal end or both. The term "derivative" is used in the present text to designate a peptide in which one or more of the amino acid residues of the peptide have been chemically modified, e.g. by alkylation, acylation, ester formation or amide formation. For the sake of simplicity, the term "peptide" will generally be used herein to refer to the glucagon and GLP-1 peptides as such as well as analogues and derivatives thereof.

The present invention is based on situations where the hydrophilic patch of the putative amphiphatic helix displays two or more charges. By the addition of a detergent carrying a complementary set of multiple charges, a stabilizing and solubilizing helix-forming effect will result from a combination of two factors, i.e. 1) charge-charge interactions between the polar head of the detergent and the charged amino acid side chains of the helix; and 2) the hydrophobic tail of the detergent serving as a template for association with the hydrophobic patch of the same or that of a neighboring helix.

The stabilized solutions of the invention can be used for parenteral, pulmonal or nasal administration. Such solutions remain stable and retain biological activity when stored at temperatures of e.g. 4°-25°C for extended periods of time.

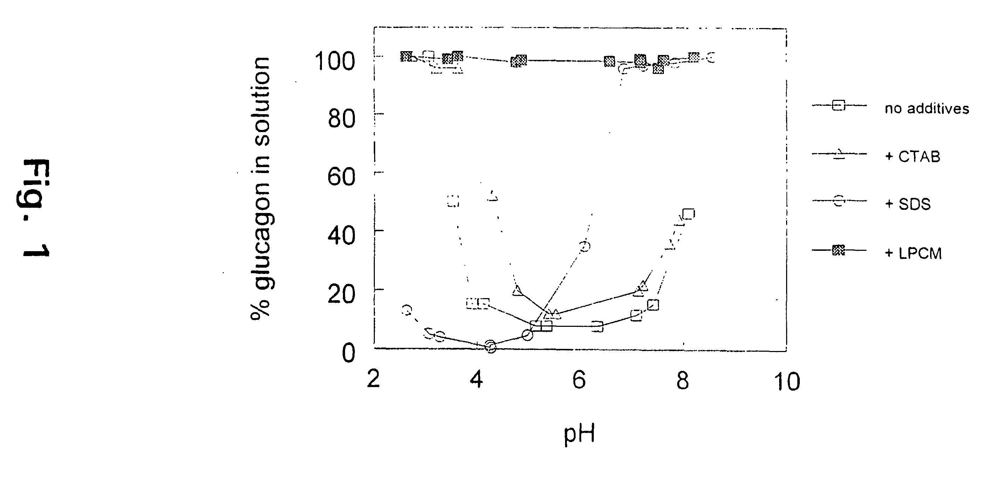

Suitable detergents according to the invention are characterized by having multiple charges, i.e. two or more positive charges, two or more negative charges, or a combination of at least one positive charge and at least one negative charge. The advantage of multiple charges as opposed to monovalent ionic detergents is illustrated in the appended Figure 1. This figure compares the pH-dependence of the equilibrium solubility in 1 mg/ml solutions of glucagon in the absence of additives and in the presence of 1 mM of each of the following detergents: a) cetyl-trimethylammonium bromide (CTAB, i.e. monovalent cationic), b) sodium dodecyl sulfate (SDS, i.e. monovalent anionic) and c) myristoyl lysophosphatidylcholine (LPCM, i.e. amphoteric).

These data show that, while the monovalent cationic and anionic detergents promote solubility at the acidic and basic limbs of the solubility curve, respectively, the amphoteric detergent effects full solubility over the entire pH range. Furthermore, the effect of the amphoteric detergent is clearly much stronger than that expected from the sum of the effects seem with each of the monovalent detergents.

The detergents used in accordance with the present invention are selected from the group consisting of 1-acyl-sn-glycero-3-phosphate esters of ethanolamine, choline, serine or threonine. Lauroyl and myristoyl derivatives of lysophosphatidylcholine, lysophosphatidylserine and lysophosphatidylthreonine are particularly useful.

An example of a suitable composition according to the invention is one comprising about 0.5-2.5 mg of peptide per ml, e.g. about 1 mg of peptide per ml, and about 1-3 mM, e.g. about 2 mM of the detergent, e.g. lauroyl or myristoyl lysophosphatidylcholine.

The stable aqueous, injectable solutions of glucagon and GLP-1 compounds according to the invention may be prepared by bringing together the peptide, water and a stabilizing and solubilizing amount of detergent. Typically, the solutions will contain other excipients to adjust ionic strength and provide buffering capacity and antibacterial action as needed. The final peptide concentration will be at least about 0.1 mg/ml, typically about 0.2-5 mg/ml, e.g. about 0.5-3 mg/ml, and the detergent is typically employed in an amount corresponding to at least about 0.3 mole of detergent per mole of peptide, more typically at least about 0.5 mole detergent/mole of peptide and up to about 40 moles detergent/mole of peptide, typically up to about 30 moles detergent/mole peptide, e.g. about 1-20 moles detergent/mole peptide, such as about 1-10 moles detergent/mole peptide. For a given peptide and detergent and a given concentration of peptide, a person skilled in the art will readily be able to determine a suitable detergent concentration that results in the desired stabilization. Isotonic ionic strength may be provided by conventional pharmaceutically acceptable agents such as NaCl, glycerol or mannitol. pH is buffered in the desired range between about 4 and about 8, typically by using acetate, citrate or phosphate buffers at 1-10 mM concentration as is well-known in the art. Finally, when a preservative is needed, appropriate antibacterial action is secured by the addition of, typically, phenol, m-cresol or benzyl alcohol or mixtures thereof.

The compositions of the invention can be used in the treatment of various diseases that can be treated by administering glucagon, GLP-1, or analogues or derivatives thereof. The particular peptide to be used and the optimal dose level for any patient will depend on the disease to be treated and on a variety of factors, including the efficacy of the specific peptide employed, the age, body weight, physical activity, and diet of the patient, on a possible combination with other drugs, and on the severity of the case. A typical dose of GLP-1 for regulation of the blood sugar level may e.g. be about 0.5-1 mg, for example once a day. A typical dose for glucagon may be approximately 1 mg, e.g. for gastro-intestinal imaging. It is recommended that the dosage of the active compound in question be determined for each individual patient in each case by those skilled in the art.

The invention will be further illustrated by the following non-limiting examples.

### EXAMPLES

### Materials & Methods

### EQUILIBRIUM SOLUBILITY

For pH-solubility profiles, solutions containing the peptide and additives (detergent and, where indicated, other excipients) at the appropriate concentrations were prepared at pH 9-10. The solutions were filtered, samples were withdrawn, and the pH was adjusted to the desired value in the range of 3-8. The samples were left for 24 hours at 23°C to attain solubility equilibrium. After centrifugation (20,000 g for 20 minutes, 23°C) of each sample, the pH was measured, and the solubility was determined from measurement of the absorbance at 276 nm of the supernatant.

### Long term physical stability

Glucagon and GLP-1 (7-37) were dissolved at twice the desired final concentration and incubated briefly (<10 minutes) at pH 11.5, 23°C before filtration and mixing with an equal volume of a filtered solution containing all the excipients in twice the desired final concentration. The pH was then measured and adjusted as needed. The solution was transferred to pen-fill cartridges containing a small glass ball (to allow visual determination of changes in solution viscosity). The containers were sealed and incubated at the desired temperature between 4°C and 37°C. At appropriate time intervals, the samples were gently turned and visually examined using a light box. When physical changes were apparent (precipitation, crystallization or gelation), the sample was centrifuged and the absorbance was measured in the supernatant to determine whether the component coming out of solution was the peptide or not.

### Example 1

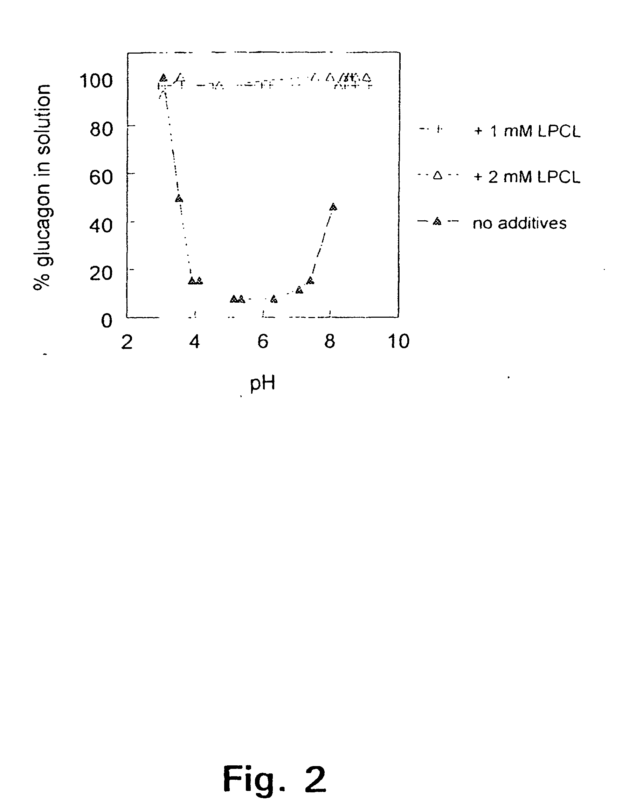

Equilibrium solubility was determined as a function of pH for 1 mg/ml glucagon solutions in the absence of additives and in the presence of 1 or 2 mM of the detergent LPCL (lauroyl lysophosphatidylcholine). The results are shown in Figure 2. It may be seen that excellent results, i.e. approximately 100% solubility throughout the pH range tested, were obtained with both concentrations of LPCL. For the control solution without additives, acceptable solubility was only obtained at pH 3.

### Example 2

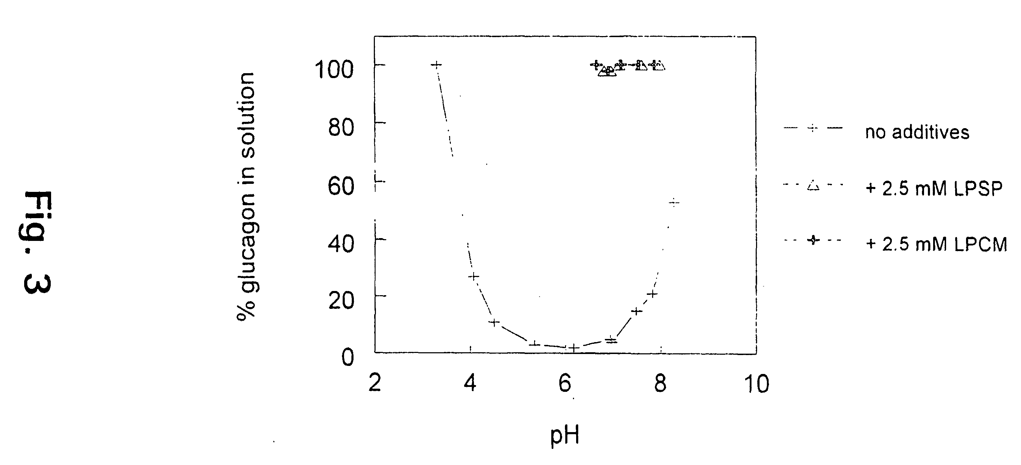

Equilibrium solubility was determined as a function of pH in the pH range of 6-8 for 2 mg/ml solutions of glucagon in the absence of additives and in the presence of 2.5 mM of the detergents LPSP (palmitoyl lysophosphatidyl-L-serine) and LPCM (myristoyl lysophosphatidyl choline). The results are shown in Figure 3. Both additives provided 100% solubility within the tested pH range of 6-8.

### Example 3

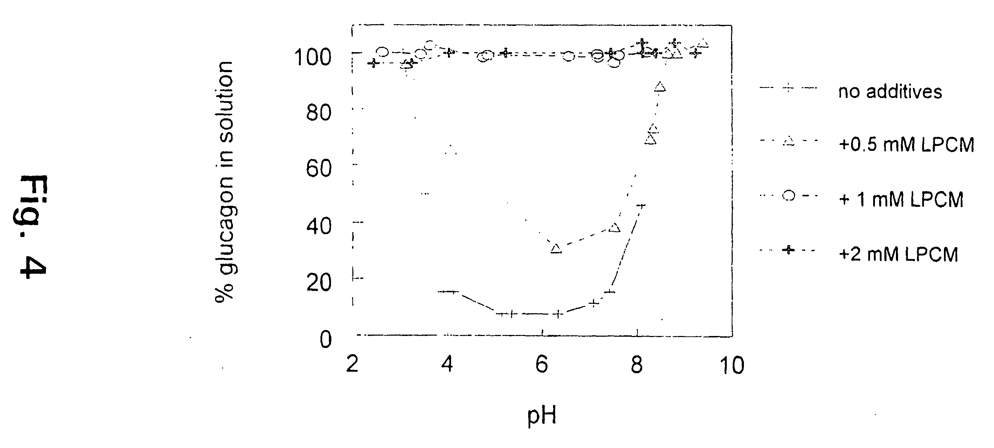

Equilibrium solubility was determined as a function of pH for 1 mg/ml solutions of glucagon in the absence of additives and in the presence of increasing amounts of LPCM (myristoyl lysophosphatidyl choline). The results are shown in Figure 4. For solutions containing 1 or 2 mM LPCM, approximately 100% solubility was obtained throughout the entire pH range, while 0.5 mM LCPM provided insufficient solubility at pH 4-8. However, even the addition of 0.5 mM LCPM gave a substantial improvement over the control solution.

### Example 4

The following examples illustrate various glucagon solutions prepared according to the invention. All of the indicated solutions remained clear, i.e. the glucagon remained completely soluble, for at least the duration of the observation period of four weeks:

| mg/ml Glucagon | mM detergent | buffer, pH | Other additive | Temperature |
|---|---|---|---|---|
| 2 | 5.0 LPCM | 5 mM phosphate, 7.2 | | 4 °C |
| 2 | 5.0 LPCM | 5 mM phosphate, 7.2 | 0.2 M mannitol | 4 °C |
| 1 | 5.0 LPCM | 5 mM phosphate, 6.0 | 0.12 M NaCl | 4 °C |
| 1 | 5.0 LPCM | 5 mM phosphate, 6.5 | 0.2 M mannitol | 4 °C |
| 1 | 2.0 LPCM | 5 mM phosphate, 7.2 | | 4 °C |
| 1 | 2.0 LPCM | 5 mM phosphate, 7.2 | 0.12 M NaCl | 4 °C |
| 1 | 2.0 LPCM | 5 mM phosphate, 6.0 | 0.12 M NaCl | 4 °C |
| 0.5 | 1.0 LPCM | 5 mM phosphate, 7.2 | 0.12 M NaCl | 4 °C |
| 0.5 | 2.0 LPCM | 5 mM phosphate, 7.2 | 0.12 M NaCl | 4 °C |
| 0.5 | 2.0 LPCM | 5 mM acetate, 4.5 | 0.12MNaCt | 4 °C |
| 0.5 | 2.0 LPCM | 5 mM acetate, 4.5 | 0.12 M NaCl | 25 °C |
| LPCM = myristoyl lysophosphatidylcholine | | | | |

| mg/ml Glucagon | mM detergent | buffer, pH | Other additive | Temperature |
|---|---|---|---|---|
| 1 | 2.5 HPS | 5 mM phosphate, 7.2 | 0.2 M mannitol | 4 °C |
| 1 | 5.0 LPCL | 5 mM phosphate, 7.0 | 0.12 M NaCl | 4 °C |
| 1 | 5.0 LPCL | 5 mM phosphate, 6.0 | 0.12 M NaCl | 4 °C |
| 1 | 2.5 LPCL | 5 mM phosphate, 7.2 | 0.2 M mannitol | 4 °C |
| 0.5 | 2.0 LPCL | 5 mM phosphate, 7.2 | 0.2 M mannitol | 4 °C |
| 0.5 | 2.0 LPCL | 5 mM phosphate, 7.2 | 0.12 M NaCl | 4 °C |
| 0.5 | 2.0 LPCL | 5 mM acetate, 5.0 | 0.2 M mannitol | 4 °C |
| 0.5 | 2.0 LPCL | 5 mM acetate, 5.0 | 0.2 M mannitol | 25 °C |
| 0.5 | 2.0 HPS | 5 mM acetate, 4.5 | 0.12 M NaCl | 25 °C |
| | | | | |
| HPS = N-Hexadecy!-N,N-dimethyl-3-ammonio-1-propanesulfonate | | | | |
| LPCL = Lauroyl lysophosphatidylcholine | | | | |

### Example 5

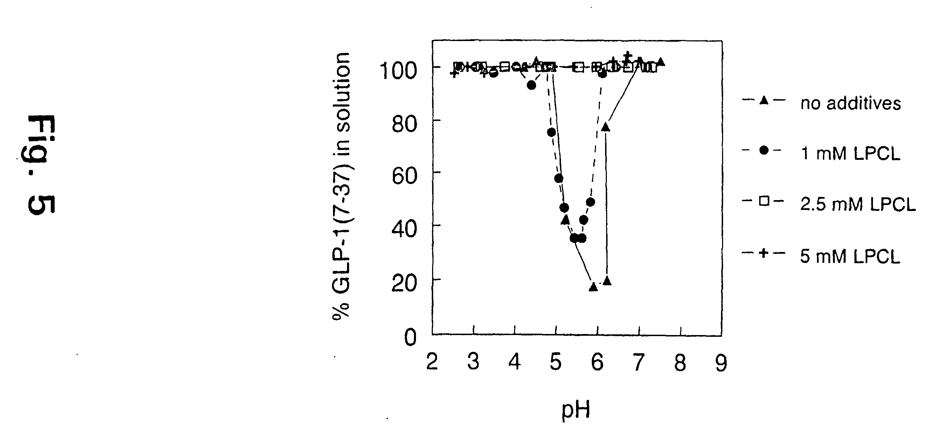

Equilibrium solubility was determined as a function of pH for 2 mg/ml solutions of GLP-1(7-37) in the absence of additives and in the presence of increasing amounts of the detergent LPCL (lauroyl lysophosphatidyl choline). The results are shown in Fig. 5. For solutions containing 2.5 and 5 mM LPCL, 100% solubility was observed throughout the entire pH range. 1.0 mM LPCL did not provide the full effect, although solubility was significantly enhanced relative to the reference composition without the detergent.

### Example 6

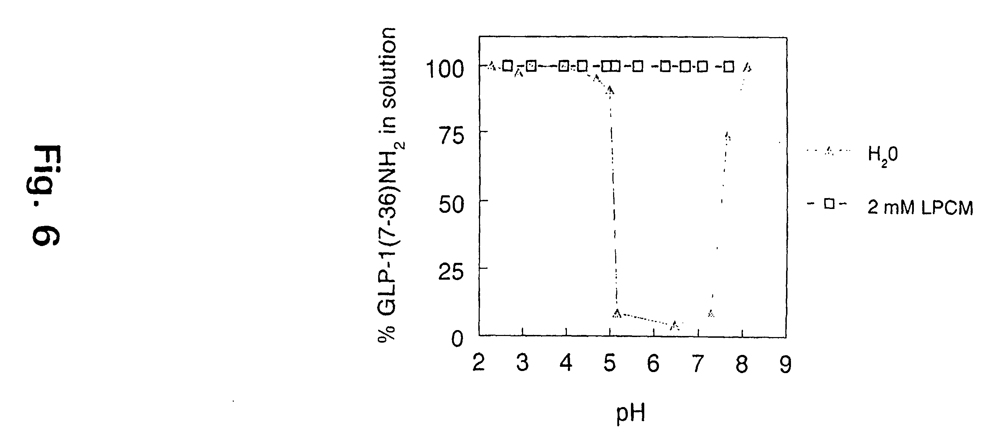

Equilibrium solubility was determined as a function of pH for 2 mg/ml solutions of GLP-1(7-36)-NH₂ in the absence of additives and in the presence of increasing amounts of the detergent LPCM (myristoyl lysophosphatidyl choline). The results shown in Fig. 6 illustrate that 2 mM LPCM provides full solubility in comparison with the reference composition.

### Example 7

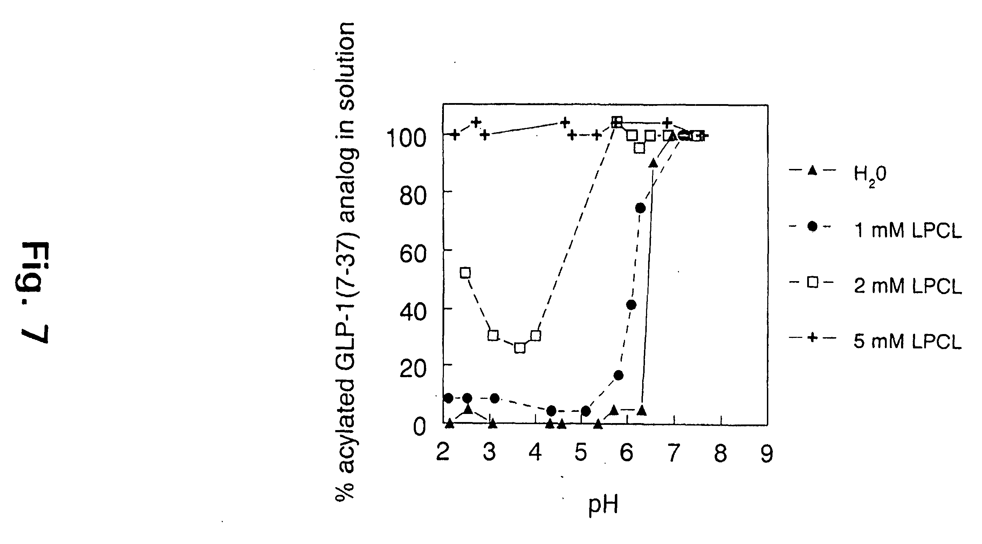

Equilibrium solubility was determined as a function of pH for 1 mg/ml solutions of the acylated GLP-1(7-37) analogue N^{ε}-hexadecanoyl-γ-glutamyl-Lys26,Arg34-GLP-1(7-37) in the absence of additives and in the presence of increasing amounts of the detergent LPCL (lauroyl lysophosphatidyl choline). The results illustrated in Fig. 7 show that 1 mM and 2 mM LPCL enhance solubility relative to the reference composition, while full solubility is obtained in the presence of 5 mM LPCL.

## Claims

1. An aqueous composition in the form of a solution comprising at least one peptide selected from glucagon, glucagon-like peptide-1 (GLP-1), and analogues and derivatives thereof, the peptide being present in a concentration of about 0.1-10 mg/ml, together with a stabilizing and solubilizing amount of at least one detergent, wherein the detergent is a 1-acyl-sn-glycero-3-phosphate ester of ethanolamine, choline, serine, or threonine or lauroyl lysophosphatidylcholine or myristoyl lysophosphatidylcholine.

2. A composition according to claim 1, wherein the peptide is present in a concentration of about 0.1-10 mg/ml, preferably about 0.2-5 mg/ml, e.g. about 0.5-3 mg/ml.

3. A composition according to claim 1 or 2, wherein the detergent is present in an amount of about 0.3-40 moles of detergent per mole of peptide, preferably about 0.5-30 moles of detergent per mole of peptide, more preferably about 1-20 moles of detergent per mole of peptide, such as about 1-10 moles of detergent per mole of peptide.

4. A composition according to claim 1 comprising about 0.5-2.5 mg of peptide per ml and about 1-3 mM of lauroyl lysophosphatidylcholine or myristoyl lysophosphatidylcholine.

5. A composition according to any of the preceding claims, further comprising an isotonic agent such as NaCl, glycerol or mannitol, a buffer substance such as acetate, citrate or phosphate buffering at pH 4-8, and, optionally, a preservative such as m-cresol, phenol or benzyl alcohol.

6. A method for stabilizing an aqueous composition in the form of a solution comprising at least one peptide selected from glucagon, glucagon-like peptide-1 (GLP-1), and analogues and derivatives thereof, wherein the peptide is present in a concentration of about 0.1-10 mg/ml, the method comprising adding to said composition a stabilising and solubilising amount of at least one detergent, wherein the at least one peptide is stabilised in the aqueous composition, and wherein the stabilising and solubilising detergent is a 1-acyl-sn-glycero-3-phosphate ester of ethanolamine, choline, serine, or threonine or lauroyl lysophosphatidylcholine or myristoyl lysophosphatidylcholine.

7. A method according to claim 6, wherein the aqueous composition is as defined in any of claims 2-5.

8. Use of an aqueous composition according to any of claims 1-5 for the preparation of a medicament for the treatment of insulin dependent or non-insulin dependent diabetes mellitus or obesity in a patient.

## Patentansprüche

1. Wäßrige Zusammensetzung in Form einer Lösung, umfassend mindestens ein Peptid, ausgewählt aus Glucagon, glucagonartigem Peptid-1 (GLP-1) und Analoga und Derivaten davon, wobei das Peptid in einer Konzentration von etwa 0,1 bis 10 mg/ml vorhanden ist, zusammen mit einer stabilisierenden und löslichmachenden Menge mindestens eines Detergens, wobei das Detergens ein 1-Acyl-sn-glycero-3-phosphatester von Ethanolamin, Cholin, Serin oder Threonin oder Lauroyllysophosphatidylcholin oder Myristoyllysophosphatidylcholin ist.

2. Zusammensetzung nach Anspruch 1, wobei das Peptid in einer Konzentration von etwa 0,1 bis 10 mg/ml, vorzugsweise etwa 0,2 bis 5 mg/ml, z.B. etwa 0,5 bis 3 mg/ml, vorhanden ist.

3. Zusammensetzung nach Anspruch 1 oder 2, wobei das Detergens in einer Menge von etwa 0,3 bis 40 Mol Detergens pro Mol Peptid, vorzugsweise etwa 0,5 bis 30 Mol Detergens pro Mol Peptid, stärker bevorzugt etwa 1 bis 20 Mol Detergens pro Mol Peptid, wie etwa 1 bis 10 Mol Detergens pro Mol Peptid, vorhanden ist.

4. Zusammensetzung nach Anspruch 1, umfassend etwa 0,5 bis 2,5 mg Peptid pro ml und etwa 1 bis 3 mM Lauroyllysophosphatidylcholin oder Myristoyllysophosphatidylcholin.

5. Zusammensetzung nach einem der vorhergehenden Ansprüche, weiter umfassend ein isotonisches Mittel, wie NaCl, Glycerin oder Mannit, eine Puffersubstanz, wie Acetat, Citrat oder Phosphat, die bei pH 4 bis 8 puffern, und gegebenenfalls ein Konservierungsmittel, wie m-Kresol, Phenol oder Benzylalkohol.

6. Verfahren zur Stabilisierung einer wäßrigen Zusammensetzung in Form einer Lösung, umfassend mindestens ein Peptid, ausgewählt aus Glucagon, glucagonartigem Peptid-1 (GLP-1) und Analoga und Derivaten davon, wobei das Peptid in einer Konzentration von etwa 0,1 bis 10 mg/ml vorhanden ist, wobei das Verfahren die Zugabe einer stabilisierenden und löslichmachenden Menge mindestens eines Detergens zu der Zusammensetzung umfaßt, wobei das mindestens eine Peptid in der wäßrigen Zusammensetzung stabilisiert ist, und wobei das stabilisierende und löslichmachende Detergens ein 1-Acyl-sn-glycero-3-phosphatester von Ethanolamin, Cholin, Serin oder Threonin oder Lauroyllysophosphatidylcholin oder Myristoyllysophosphatidylcholin ist.

7. Verfahren nach Anspruch 6, wobei die wäßrige Zusammensetzung wie in einem der Ansprüche 2 bis 5 definiert ist.

8. Verwendung einer wäßrigen Zusammensetzung nach einem der Ansprüche 1 bis 5 zur Herstellung eines Medikaments zur Behandlung von insulinabhängigem oder nichtinsulinabhängigem Diabetes mellitus oder Obesität bei einem Patienten.

## Revendications

1. Composition aqueuse sous la forme d'une solution comprenant au moins un peptide choisi parmi le glucagon, le peptide-1 de type glucagon (GLP-1), et des analogues et dérivés de ceux-ci, le peptide étant présent en une concentration d'environ 0,1 à 10 mg/ml, conjointement avec une quantité stabilisante et solubilisante d'au moins un détergent, dans laquelle le détergent est un ester 1-acyl-sn-glycéro-3-phosphate d'éthanolamine, de choline, de sérine ou de thréonine ou la lauroyl lysophosphatidylcholine ou la myristoyl lysophosphatidylcholine.

2. Composition selon la revendication 1, dans laquelle le peptide est présent en une concentration d'environ 0,1 à 10 mg/ml, de préférence d'environ 0,2 à 5 mg/ml, par exemple d'environ 0,5 à 3 mg/ml.

3. Composition selon la revendication 1 ou la revendication 2, dans laquelle le détergent est présent en une quantité d'environ 0,3 à 40 moles de détergent par mole de peptide, de préférence d'environ 0,5 à 30 moles de détergent par mole de peptide, de manière davantage préférée d'environ 1 à 20 moles de détergent par mole de peptide, comme par exemple d'environ 1 à 10 moles de détergent par mole de peptide.

4. composition selon la revendication 1, comprenant d'environ 0,5 à 2,5 mg de peptide par ml et environ 1 à 3 mM de lauroyl lysophosphatidylcholine ou de myristoyl lysophosphatidylcholine.

5. Composition selon l'une quelconque des revendications précédentes, comprenant en outre un agent isotonique tel que le NaCl, le glycerol ou le mannitol, une substance tampon telle qu'un tampon acétate, citrate ou phosphate à pH 4 à 8, et, facultativement, un conservateur tel que le m-crésol, le phénol ou l'alcool benzylique.

6. Procédé de stabilisation d'une composition aqueuse sous la forme d'une solution comprenant au moins un peptide choisi parmi le glucagon, le peptide-1 de type glucagon (GLP-1), et des analogues et dérivés de ceux-ci, dans lequel le peptide est présent en une concentration d'environ 0,1 à 10 mg/ml, le procédé comprenant l'addition à ladite composition d'une quantité stabilisante et solubilisante d'au moins un détergent, dans lequel le au moins un peptide est stabilisé dans la composition aqueuse, et dans lequel le détergent stabilisant et solubilisant est un ester 1-acyl-sn-glycéro-3-phosphate d'éthanolamine, de choline, de sérine ou de thréonine ou la lauroyl lysophosphatidylcholine ou la myristoyl lysophosphatidylcholine.

7. Procédé selon la revendication 6, dans lequel la composition aqueuse est telle que définie dans l'une quelconque des revendication 2 à 5.

8. Utilisation d'une composition aqueuse selon l'une quelconque des revendications 1 à 5 pour la préparation d'un médicament destiné au traitement du diabète sucré insulinodépendant ou non insulinodépendant ou de l'obésité chez un patient.
